# EUROPEAN PATENT APPLICATION

(11) **EP 4 502 142 A1**
(43) Date of publication of application: **05.02.2025**
(21) Application number: 23779128.0
(22) Date of filing: 28.02.2023
(51) Int. Cl.: C12N 5/07, A61B 3/10

(54) **METHOD FOR DETERMINING PROPERTIES OF LIGHT**

(30) Priority: 30.03.2022 JP 2022056705
(71) Applicant: Tokyo Metropolitan Public University Corporation, Shinjuku-ku Tokyo 163-0926 (JP); HOYA LENS THAILAND LTD., Pathumthani 12130 (TH)
(72) Inventor: MIYOSHI, Hiromi, Hachioji-shi, Tokyo 192-0397 (JP); IGUCHI, Yuki, Tokyo 160-8347 (JP)
(74) Representative: Betten & Resch
(86) International application number: PCT/JP2023/007185
(87) International publication number: WO 2023/189098

(57) **Abstract**

A method of determining characteristics of light, including: a relationship acquisition step of, with respect to a subject, obtaining in advance a relationship 1 between a wavelength and irradiance of light that decides whether or not a morphological change of an ocular cell is caused and a relationship 2 between the wavelength and irradiance of the light that decides whether or not migration of the ocular cell is changed; a relationship selection step of selecting at least any of the relationship 1 and the relationship 2 depending on a degree 1 that is required for the morphological change and a degree 2 that is required for the cell migration; and a determination step of determining a wavelength and irradiance of light to be incident on the subject's eye based on the selected relationship.

## Description

### [Technical Field]

The present invention relates to a method of determining the characteristics of light.

### [Background Art]

PTL 1 describes the influence of light on retinal pigment epithelial cells (RPE). RPE apoptosis is described to be extremely induced in 10 nm bandwidths having 420, 430, 440, or 450 nm (415 to 455 nm) at the center. In addition, prevention of the transmission of light having a wavelength in a harmful bandwidth with selective filtering means is proposed.

### [Citation List]

### [Patent Literature]

[PTL 1] Japanese Patent No. 6773391

### [Summary of Invention]

### [Technical Problem]

The present inventors studied symptoms, diseases and the like that are caused by an abnormality in a lens epithelial cell such as a secondary cataract. The secondary cataract is as described below.

One example of a treatment for patients suffering from cataracts is a cataract surgery. Ordinarily, a crystalline lens that has become cloudy is removed from the inside of the incised lens capsule (phacoemulsification), and an intraocular lens is inserted instead into the lens capsule. The intraocular lens is covered with the partially-incised lens capsule.

After the surgery, complications may occur. As one of the complications, there are cases where, after the surgery, the lens epithelial cell or the like that remains in the lens capsule proliferates in the gap between the rear surface (the surface in the eye that is opposite to an object in front of the eye) of the intraocular lens and the lens capsule, whereby the lens capsule becomes cloudy (posterior capsule opacification), the posterior capsule opacification further progresses to lower the translucency into the eye, and the visual function deteriorates, which is referred to as "secondary cataract." A typical treatment method in this case is the removal of turbidity using a YAG radar.

In Japan, around the year of 2020, surgeries to treat secondary cataracts accounted for about 30% of all cataract surgeries (lens reconstruction surgeries), which has been considered as a problem. In addition, abnormalities of the lens epithelial cell are also considered to relate to other symptoms and diseases regarding crystalline lenses.

Regarding the lens epithelial cells, which are one cause of secondary cataracts, the present inventors found that it is extremely meaningful to learn what characteristics of light that is incident in the living environment of each individual (hereinafter, also referred to as "subject"), that is, on the subject's eye cause a change in the lens epithelial cell or how the lens epithelial cell changes.

Based on this information, the present inventors investigated the influence of the characteristics (for example, the wavelength and irradiance of light) of light that enters an eye on the lens epithelial cells. As a result, it was found that, depending on the characteristics of light that enters the eye, a morphological change or irregular morphology can be caused in the lens epithelial cell, and the migration of the lens epithelial cell can be changed (for example, degraded). Furthermore, it was found that the information can also be applied to eye-related cells other than the lens epithelial cell (hereinafter, referred to as "ocular cells"; specific examples of the ocular cells will be described below).

An object of one embodiment of the present invention is to determine the characteristics of light that is made incident on an eye based on the relation between the morphological change and a change in the migration of the ocular cells and the characteristics of light.

### [Solution to Problem]

It is meaningful that a subject ascertains in advance what characteristics of light that enters an eye causes a morphological change in the ocular cells and what characteristics of light that enters an eye causes a change in the migration of the ocular cells.

As a result of conducting additional intensive studies based on each information described above, an idea of means for determining what characteristics light to be made incident on the subject's eye needs to have was obtained. What has been created based on this information is the following aspects.

A first aspect of the present invention is a method of determining characteristics of light, including:
a relationship acquisition step of, with respect to a subject, obtaining in advance a relationship 1 between a wavelength and irradiance of light that decides whether or not a morphological change of an ocular cell is caused and a relationship 2 between the wavelength and irradiance of the light that decides whether or not migration of the ocular cell is changed,
a relationship selection step of selecting at least any of the relationship 1 and the relationship 2 depending on a degree 1 that is required for the morphological change and a degree 2 that is required for the cell migration, and
a determination step of determining a wavelength and irradiance of light to be incident on the subject's eye based on the selected relationship.

A second aspect of the present invention is the method of determining characteristics of light according to the first aspect,
in which the relationship 1 is a plot 1 that decides whether or not a morphological change of the ocular cell is caused in a relation between the wavelength and irradiance of the light, and
the relationship 2 is a plot 2 that decides whether or not the migration of the ocular cell is changed in the relation between the wavelength and irradiance of the light.

A third aspect of the present invention is the method of determining characteristics of light according to the second aspect,
in which, in the relation between the wavelength and irradiance of the light, the plot 1 is present on a short wavelength side of the plot 2,
in the relation between the wavelength and irradiance of the light, the plot 1 classifies a region into a plurality of regions 1 composed of a region where the morphological change is caused and a region where the morphological change is not caused,
in the relation between the wavelength and irradiance of the light, the plot 2 classifies a region into a plurality of regions 2 composed of a region where a change of the cell migration is caused and a region where a change of the cell migration is not caused,
in the relationship selection step, the plot 1 and the plot 2 are selected,
a region selection step of selecting one region from the regions 1 and selecting one region from the regions 2 is further provided after the relationship selection step,
in the relationship selection step, at least (1) of the following (1) and (2) is performed: (1) the region where the morphological change is not caused is selected from the regions 1, and
(2) the region where a change in the cell migration is not caused is selected from the regions 2, and
in the determination step, a wavelength and irradiance of light commonly belonging to the individual selected regions are obtained as the wavelength and irradiance of the light that is to be incident on the subject's eyes.

A fourth aspect of the present invention is the method of determining characteristics of light according to the third aspect,
in which, in addition to the plot 2, a plot 3 that further classifies the region where a change of the cell migration is caused in the regions 2 depending on a degree of the change of the cell migration is further obtained in the relationship acquisition step.

A fifth aspect of the present invention is the method of determining characteristics of light according to any one of the second to fourth aspects,
in which the plots 1 and 2 are created based on regional information where the subject resides.

A sixth aspect of the present invention is the method of determining characteristics of light according to any one of the first to fifth aspects,
in which the ocular cell is a lens epithelial cell, a cell retinal pigment epithelial cell, a corneal endothelial cell, a corneal epithelial stem cell, or a subconjunctival fibroblast.

A seventh aspect of the present invention is a method of determining characteristics of light including:
a relationship acquisition step of, with respect to a subject, obtaining in advance at least one of
   · a relationship 1 between a wavelength and irradiance of light that decides whether or not a morphological change of an ocular cell is caused,
   · a relationship 2 between the wavelength and irradiance of the light that decides whether or not migration of the ocular cell is changed, and
   · a relationship 3 between the wavelength and irradiance of the light that decides whether or not invasion of the ocular cell is increased;
a relationship selection step of selecting at least any of the relationship 1, the relationship 2, and the relationship 3 depending on at least any of the degree 1 that is required for the morphological change, the degree 2 that is required for the cell migration, and the degree 3 that is required for the invasion; and
a determination step of determining the wavelength and irradiance of light that is to be incident on the subject's eyes based on the selected relationship.

An eighth aspect of the present invention is the method of determining characteristics of light according to the seventh aspect,
in which the relationship 1 is a plot 1 that decides whether or not a morphological change of the ocular cell is caused in a relation between the wavelength and irradiance of the light,
the relationship 2 is a plot 2 that decides whether or not the migration of the ocular cell is changed in the relation between the wavelength and irradiance of the light, and
the relationship 3 is a plot 4 that decides whether or not the invasion of the ocular cell is increased in the relation between the wavelength and irradiance of the light.

A ninth aspect of the present invention is the method of determining characteristics of light according to the seventh or eighth aspect,
in which, in the relationship acquisition step, at least the relationship 3 is obtained in advance, and in the relationship selection step, at least the relationship 3 is selected.

A tenth aspect of the present invention is the method of determining characteristics of light according to the seventh or eighth aspect,
in which, in the relationship acquisition step, the relationships 1 to 3 are obtained in advance, and in the relationship selection step, the relationships 1 to 3 are selected.

An eleventh aspect of the present invention is the method of determining characteristics of light according to the tenth aspect,
in which, in the relation between the wavelength and irradiance of the light, the plot 1 is present on a short wavelength side of the plot 2,
in the relation between the wavelength and irradiance of the light, the plot 1 classifies a region into a plurality of regions 1 composed of a region where the morphological change is caused and a region where the morphological change is not caused,
in the relation between the wavelength and irradiance of the light, the plot 2 classifies a region into a plurality of regions 2 composed of a region where a change of the cell migration is caused and a region where a change of the cell migration is not caused,
in the relation between the wavelength and irradiance of the light, the plot 4 classifies a region into a plurality of regions 4 composed of a region where an increase of the invasion is caused and a region where an increase of the invasion is not caused,
in the relationship selection step, the plot 1, the plot 2, and the plot 4 are selected,
a region selection step of selecting one region from the regions 1, selecting one region from the regions 2, and selecting one region from the regions 4 is further provided after the relationship selection step, and
in the relationship selection step, at least (1) of the following (1) to (3) is performed:
   (1) the region where the morphological change is not caused is selected from the regions 1,
   (2) the region where a change in the cell migration is not caused is selected from the regions 2, and
   (3) the region where an increase of the invasion is not caused is selected from the regions 4, and
in the determination step, a wavelength and irradiance of light commonly belonging to the individual selected regions are obtained as the wavelength and irradiance of light that is to be incident on the subject's eyes.

A twelfth aspect of the present invention is the method of determining characteristics of light according to the eleventh aspect,
in which, in addition to the plot 2, a plot 3 that further classifies the region where a change of the cell migration is caused in the regions 2 depending on a degree of the change of the cell migration is further obtained in the relationship acquisition step.

A thirteenth aspect of the present invention is the method of determining characteristics of light according to the twelfth aspect,
in which, in the relation between the wavelength and irradiance of the light, the plot 4 is on the short wavelength side of the plot 3 and is on the long wavelength side of the plot 2.

A fourteenth aspect of the present invention is the method of determining characteristics of light according to the tenth aspect,
in which at least the plots 1, 2 and 4 are created based on regional information where the subject resides.

A fifteenth aspect of the present invention is the method of determining characteristics of light according to any one of the seventh to fourteenth aspect,
in which the ocular cell is a lens epithelial cell, a cell retinal pigment epithelial cell, a corneal endothelial cell, a corneal epithelial stem cell, or a subconjunctival fibroblast.

### [Advantageous Effects of Invention]

According to one example of the present invention, it is possible to determine the characteristics of light based on the relation between the morphological change and a change in the migration of the ocular cell and the characteristics of light.

### [Brief Description of Drawings]

[Fig. 1]

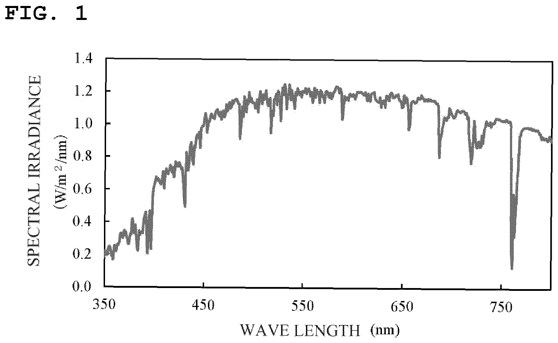

   Fig. 1 is a plot showing the spectral irradiance (vertical axis: the unit is W/m²/nm) and wavelength (horizontal axis: the unit is nm) of sunlight (AM1.5 and after corneal penetration) on the front surface of a crystalline lens.
[Fig. 2]

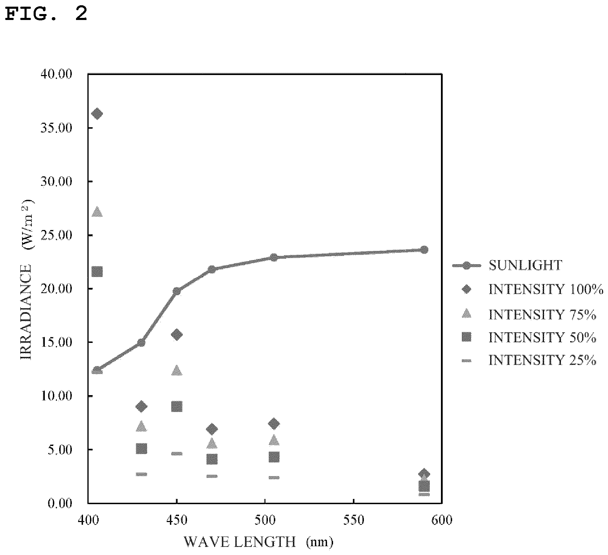

   Fig. 2 is a plot showing the irradiance (vertical axis: the unit is W/m²) and wavelength (horizontal axis: the unit is nm) of each LED light (half width: about 20 nm) used in experiments. Sunlight on the front surface of the crystalline lens for each bandwidth under the same conditions is also shown in the plots for reference.
[Fig. 3]

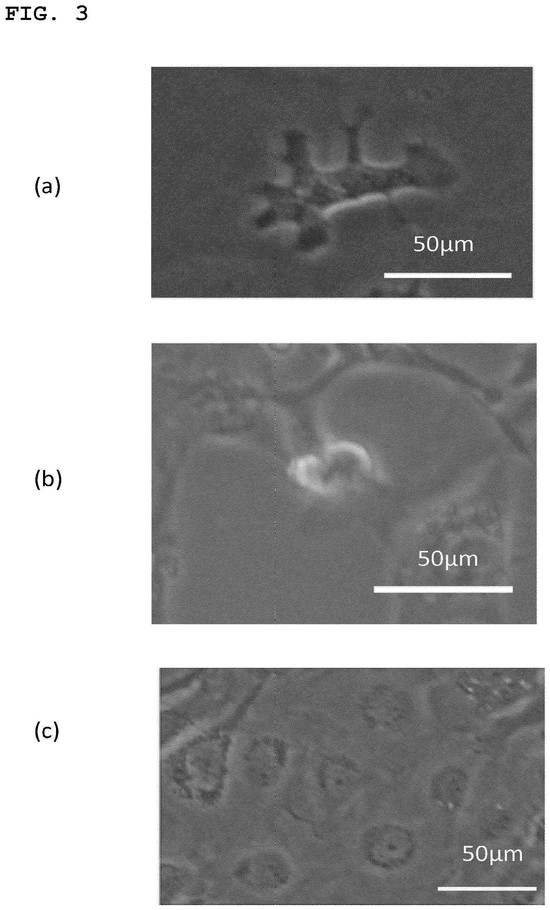

   Fig. 3 is photographs showing the observation results in the qualitative evaluation. The light irradiation conditions in Fig. 3(a) are a wavelength of 430 nm and an irradiance of 9.0 W/m², the light irradiation conditions in Fig. 3(b) are a wavelength of 430 nm and an irradiance of 5.1 W/m², and the light irradiation conditions in Fig. 3(c) are a wavelength of 450 nm and an irradiance of 4.6 W/m².
[Fig. 4]

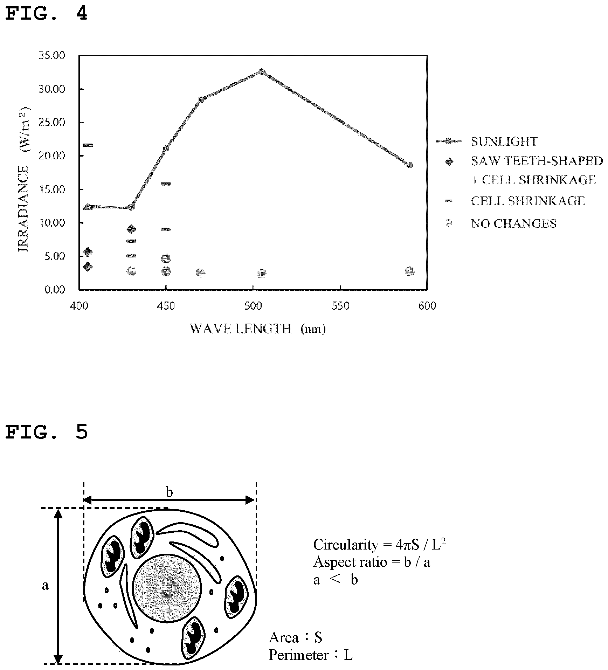

   Fig. 4 is a plot in which the morphological change of the lens epithelial cell is summarized by aspect in the relation between the irradiance (vertical axis: the unit is W/m²) and the wavelength (horizontal axis: the unit is nm) of each LED light (half width of about 20 nm) used in the experiment. Sunlight on the front surface of the crystalline lens for each bandwidth under the same conditions is also shown in the plots for reference.
[Fig. 5]
   Fig. 5 is a view for describing the circularity and aspect ratio of the lens epithelial cell. When the area is denoted as S, and the circumferential length is denoted as L, the circularity is indicated by 2πS/L² in the present specification. When the major axis of the lens epithelial cell elliptically approximated with ImageJ is denoted as a, and the minor axis is denoted as b, the aspect ratio is indicated by a/b in the present specification.
[Fig. 6]

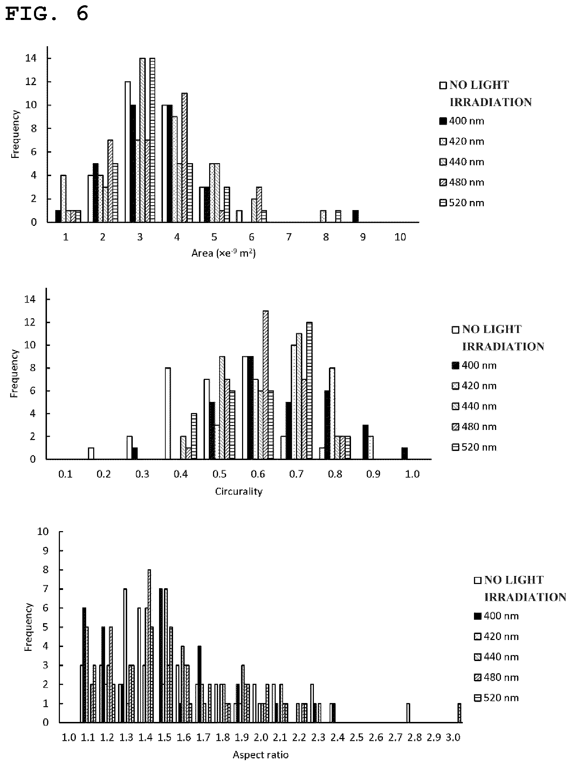

   Fig. 6 is bar graphs showing the results of the quantitative evaluation, Fig. 6(a) is a graph of the area S, Fig. 6(b) is a graph of the circularity, and Fig. 6(c) is a graph of the aspect ratio.
[Fig. 7]

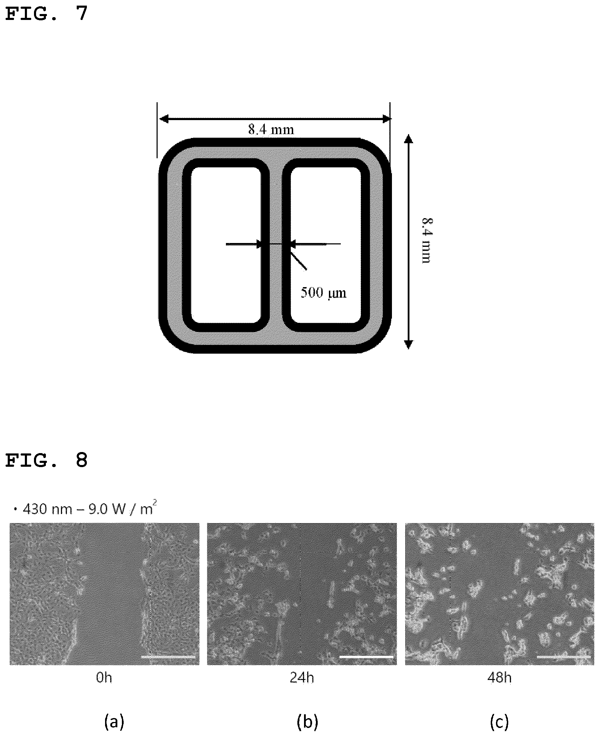

   Fig. 7 is a schematic plan view of wells used to form a gap (scratch) having a predetermined width on a surface having a large number of cells.
[Fig. 8]
   Fig. 8 is views showing the appearances of the blocking of the gap when the wavelength was 430 nm and the irradiance was 9.0 W/m², Fig. 8(a) is a photograph showing the appearance of the gap after 0 hours (immediately after the initiation of the light irradiation), Fig. 8(b) is a photograph showing the appearance of the gap after 24 hours from the initiation of the light irradiation, and Fig. 8(c) is a photograph showing the appearance of the gap after 48 hours from the initiation of the light irradiation.
[Fig. 9]

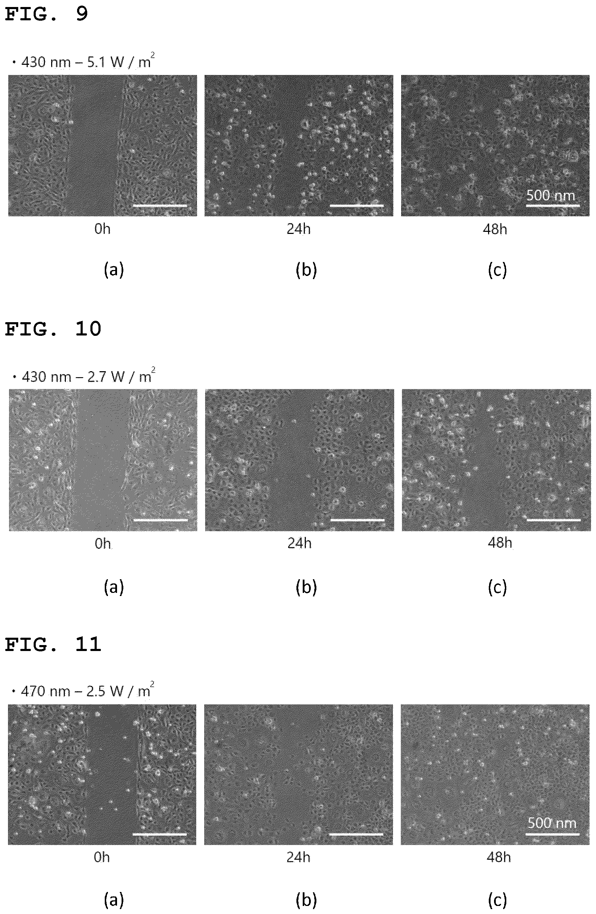

   Fig. 9 is views showing the appearances of the blocking of the gap when the wavelength was 430 nm and the irradiance was 5.1 W/m², Fig. 9(a) is a photograph showing the appearance of the gap after 0 hours (immediately after the initiation of the light irradiation), Fig. 9(b) is a photograph showing the appearance of the gap after 24 hours from the initiation of the light irradiation, and Fig. 9(c) is a photograph showing the appearance of the gap after 48 hours from the initiation of the light irradiation.
[Fig. 10]
   Fig. 10 is views showing the appearances of the blocking of the gap when the wavelength was 430 nm and the irradiance was 2.7 W/m², Fig. 10(a) is a photograph showing the appearance of the gap after 0 hours (immediately after the initiation of the light irradiation), Fig. 10(b) is a photograph showing the appearance of the gap after 24 hours from the initiation of the light irradiation, and Fig. 10(c) is a photograph showing the appearance of the gap after 48 hours from the initiation of the light irradiation.
[Fig. 11]
   Fig. 11 is views showing the appearances of the blocking of the gap when the wavelength was 470 nm and the irradiance was 2.5 W/m², Fig. 11(a) is a photograph showing the appearance of the gap after 0 hours (immediately after the initiation of the light irradiation), Fig. 11(b) is a photograph showing the appearance of the gap after 24 hours from the initiation of the light irradiation, and Fig. 11(c) is a photograph showing the appearance of the gap after 48 hours from the initiation of the light irradiation.
[Fig. 12]

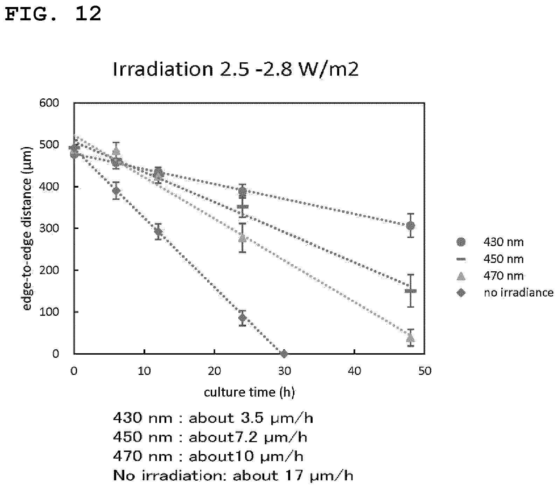

   Fig. 12 is a plot in which the relation between the distance (unit: µm) between the edges of the gap and the time from the initiation of the light irradiation (horizontal axis: the unit is h) is summarized for each wavelength. The slope of the plot is distance/time and indicates the migration rate of the lens epithelial cell.
[Fig. 13]

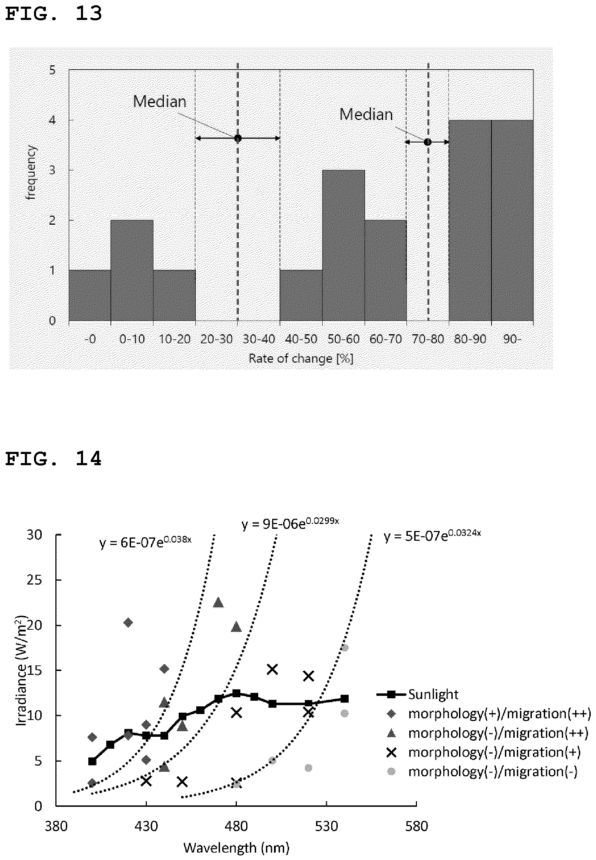

   Fig. 13 is a histogram showing the distribution of the decrease rates of the cell migration during the gap blocking.
[Fig. 14]
   Fig. 14 is a plot in which the aspects of the morphological changes and the aspects of the cell migration changes of lens epithelial cells are grouped together in the relation between the irradiance (vertical axis: the unit is W/m²) and wavelength (horizontal axis: the unit is nm) of each light source (half width: 10 nm) used in the experiment. Sunlight is also shown in the plots for reference.
[Fig. 15]

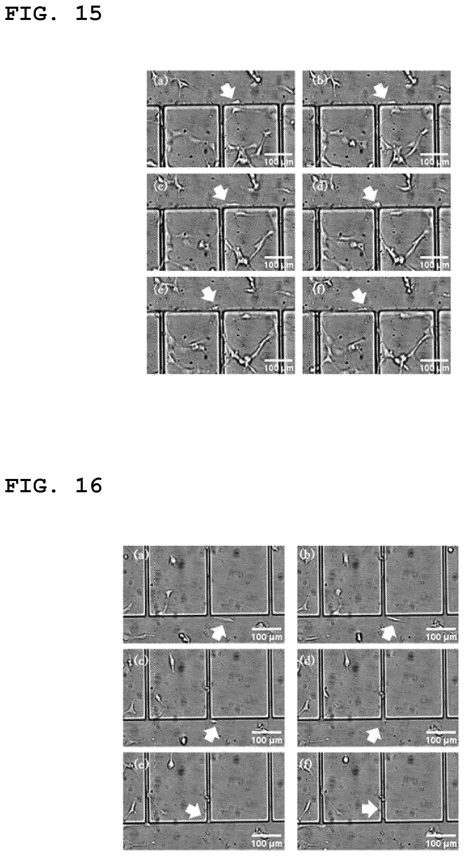

   Fig. 15 is plan views showing the appearances of the ocular cells passing through the opening portions at the time of no light irradiation NC, Fig. 15(a) is a photograph showing the appearance after 0 hours (immediately after the initiation of light irradiation), Fig. 15(b) is a photograph showing the appearance after 10 minutes from the initiation of light irradiation, Fig. 15(c) is a photograph showing the appearance after 20 minutes from the initiation of light irradiation, Fig. 15(d) is a photograph showing the appearance after 30 minutes from the initiation of light irradiation, Fig. 15(e) is a photograph showing the appearance after 40 minutes from the initiation of light irradiation, and Fig. 15(f) is a photograph showing the appearance after 50 minutes from the initiation of light irradiation.
[Fig. 16]
   Fig. 16 is plan views showing the appearances of the ocular cells invading (into the grooves) at the time of no light irradiation NC, Fig. 16(a) is a photograph showing the appearance after 0 hours (immediately after the initiation of light irradiation), Fig. 16(b) is a photograph showing the appearance after 10 minutes from the initiation of light irradiation, Fig. 16(c) is a photograph showing the appearance after 20 minutes from the initiation of light irradiation, Fig. 16(d) is a photograph showing the appearance after 30 minutes from the initiation of light irradiation, Fig. 16(e) is a photograph showing the appearance after 40 minutes from the initiation of light irradiation, and Fig. 16(f) is a photograph showing the appearance after 50 minutes from the initiation of light irradiation.
[Fig. 17]

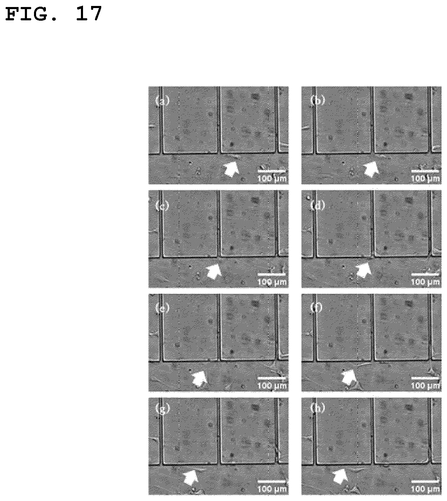

   Fig. 17 is plan views showing the appearances of the ocular cells performing the incomplete invasion behavior (failed invasion) at the time of no light irradiation NC, Fig. 17(a) is a photograph showing the appearance after 0 hours (immediately after the initiation of light irradiation), Fig. 17(b) is a photograph showing the appearance after 10 minutes from the initiation of light irradiation, Fig. 17(c) is a photograph showing the appearance after 20 minutes from the initiation of light irradiation, Fig. 17(d) is a photograph showing the appearance after 30 minutes from the initiation of light irradiation, Fig. 17(e) is a photograph showing the appearance after 40 minutes from the initiation of light irradiation, Fig. 17(f) is a photograph showing the appearance after 50 minutes from the initiation of light irradiation, Fig. 17(g) is a photograph showing the appearance after 60 minutes from the initiation of light irradiation and Fig. 17(h) is a photograph showing the appearance after 70 minutes from the initiation of light irradiation.
[Fig. 18]

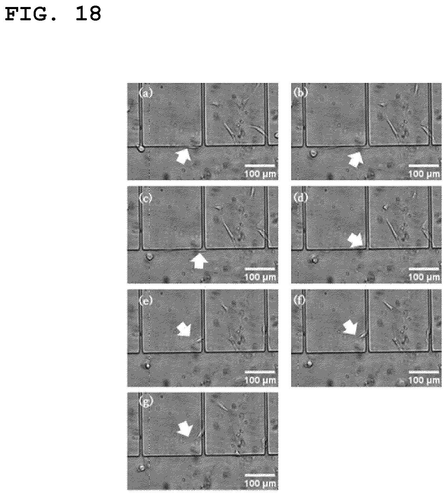

   Fig. 18 is plan views showing the appearances of the ocular cells invading (and crawling from the grooves) at the time of no light irradiation NC, Fig. 18(a) is a photograph showing the appearance after 0 hours (immediately after the initiation of light irradiation), Fig. 18(b) is a photograph showing the appearance after 10 minutes from the initiation of light irradiation, Fig. 18(c) is a photograph showing the appearance after 20 minutes from the initiation of light irradiation, Fig. 18(d) is a photograph showing the appearance after 30 minutes from the initiation of light irradiation, Fig. 18(e) is a photograph showing the appearance after 40 minutes from the initiation of light irradiation, Fig. 18(f) is a photograph showing the appearance after 50 minutes from the initiation of light irradiation and Fig. 18(g) is a photograph showing the appearance after 60 minutes from the initiation of light irradiation.
[Fig. 19]

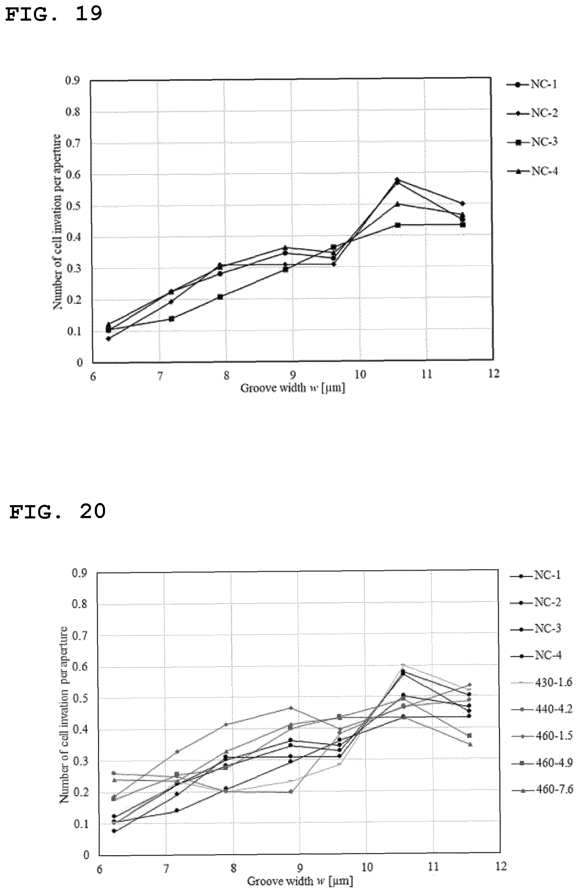

   Fig. 19 is a view showing plots NC-1, NC-2, NC-3 and NC-4 wherein the number of cells invading (into grooves) is indicated along the vertical axis and the width w (µm) of the groove is indicated along the horizontal axis.
[Fig. 20]
   Fig. 20 is a view showing the plots NC-1, NC-2, NC-3 and NC-4 and the results at wavelengths of 430 to 460 nm when the number of cells invading (into grooves) is indicated along the vertical axis and the width w (µm) of the groove is indicated along the horizontal axis.
[Fig. 21]

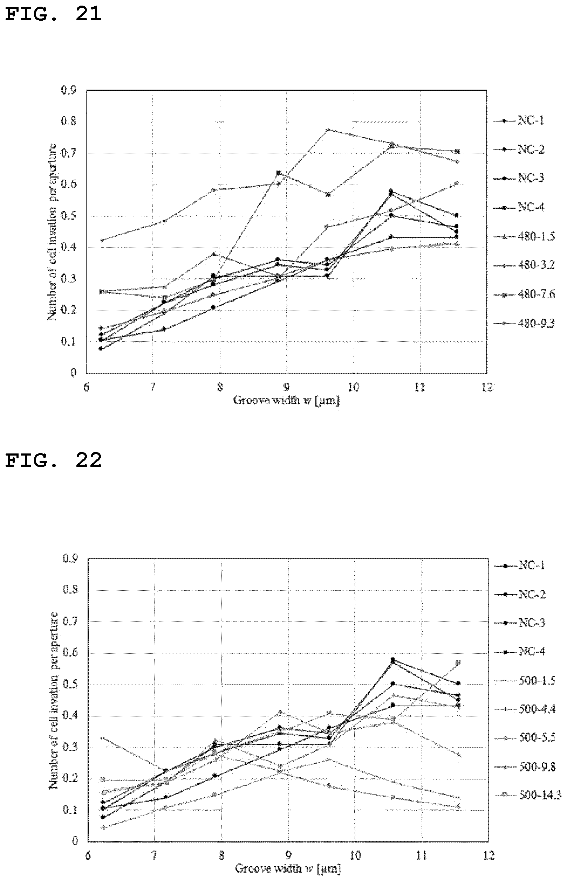

   Fig. 21 is a view showing the plots NC-1, NC-2, NC-3 and NC-4 and the results at a wavelength of 480 nm when the number of cells invading (into grooves) is indicated along the vertical axis and the width w (µm) of the groove is indicated along the horizontal axis.
[Fig. 22]
   Fig. 22 is a view showing the plots NC-1, NC-2, NC-3 and NC-4 and the results at a wavelength of 500 nm when the number of cells invading (into grooves) is indicated along the vertical axis and the width w (µm) of the groove is indicated along the horizontal axis.
[Fig. 23]

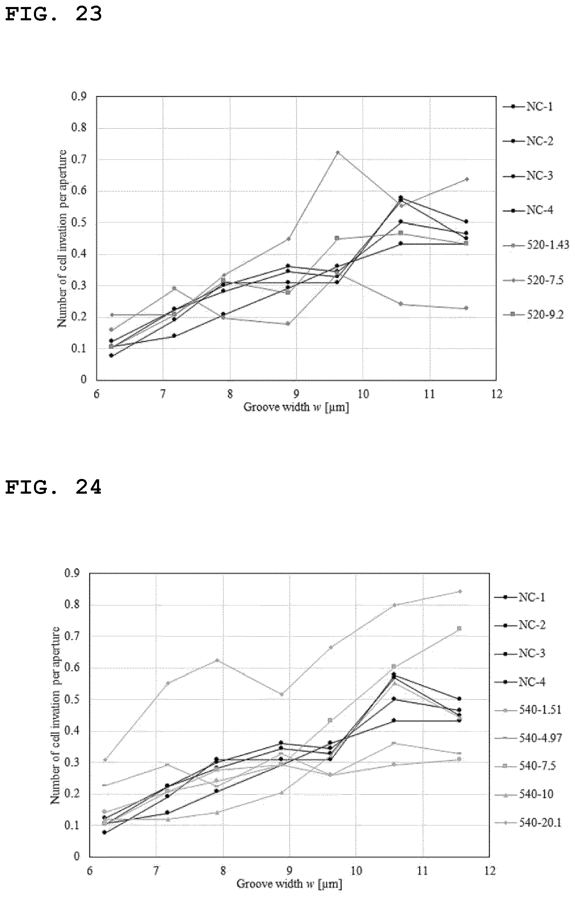

   Fig. 23 is a view showing the plots NC-1, NC-2, NC-3 and NC-4 and the results at a wavelength of 520 nm when the number of cells invading (into grooves) is indicated along the vertical axis and the width w (µm) of the groove is indicated along the horizontal axis.
[Fig. 24]
   Fig. 24 is a view showing the plots NC-1, NC-2, NC-3 and NC-4 and the results at a wavelength of 540 nm when the number of cells invading (into grooves) is indicated along the vertical axis and the width w (µm) of the groove is indicated along the horizontal axis.
[Fig. 25]

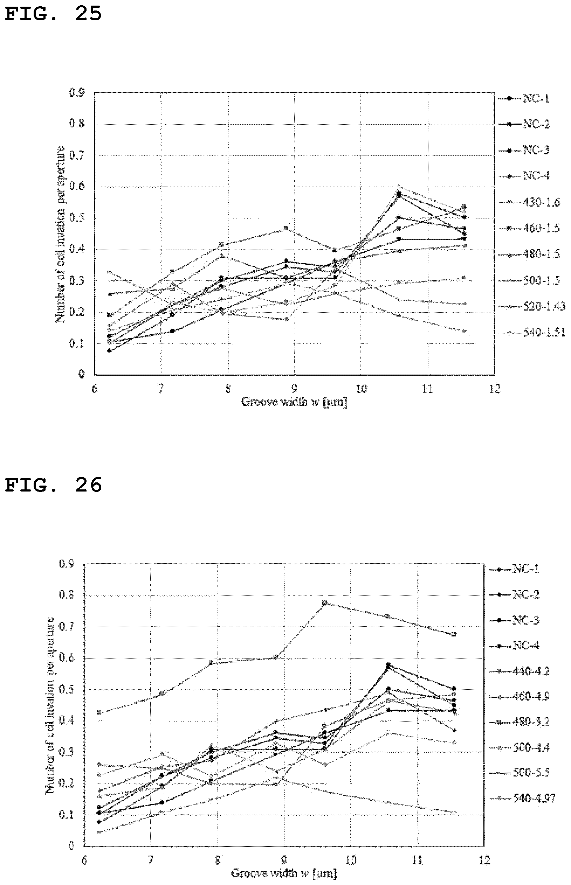

   Fig. 25 is a view showing the results when the number of cells invading (into grooves) is indicated along the vertical axis and the width w (µm) of the groove is indicated along the horizontal axis in the case of low irradiance of 1.4 to 1.5 W/m².
[Fig. 26]
   Fig. 26 is a view showing the results when the number of cells invading (into grooves) is indicated along the vertical axis and the width w (µm) of the groove is indicated along the horizontal axis in the case of intermediate irradiance of 3.2 to 5.5 W/m².
[Fig. 27]

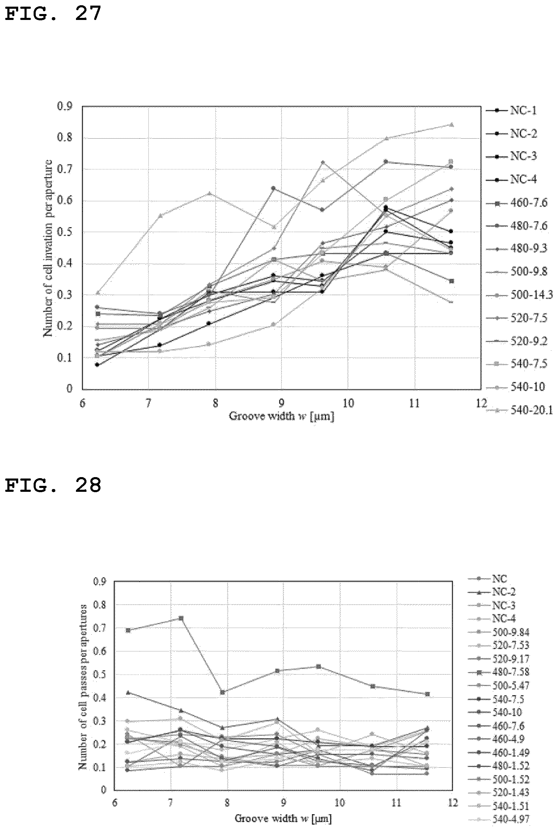

   Fig. 27 is a view showing the results when the number of cells invading (into grooves) is indicated along the vertical axis and the width w (µm) of the groove is indicated along the horizontal axis in the case of high irradiance of 7.5 W/m² or more.
[Fig. 28]
   Fig. 28 is a view showing the results when the number of cells passing is indicated along the vertical axis and the width w (µm) of the groove is indicated along the horizontal axis.
[Fig. 29]

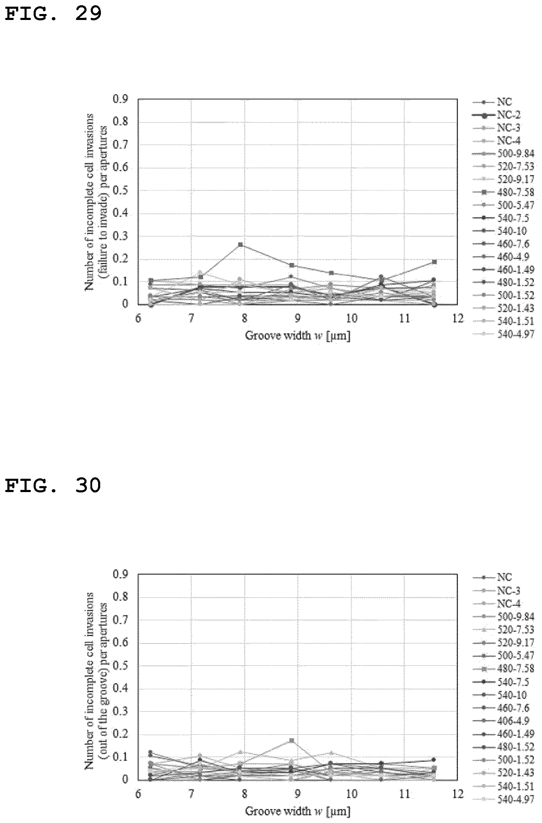

   Fig. 29 is a view showing the results when the number of cells of incomplete invasion (failed invasion) is indicated along the vertical axis and the width w (µm) of the groove is indicated along the horizontal axis.
[Fig. 30]
   Fig. 30 is a view showing the results when the number of cells invading (crawling from grooves) is indicated along the vertical axis and the width w (µm) of the groove is indicated along the horizontal axis.
[Fig. 31A]

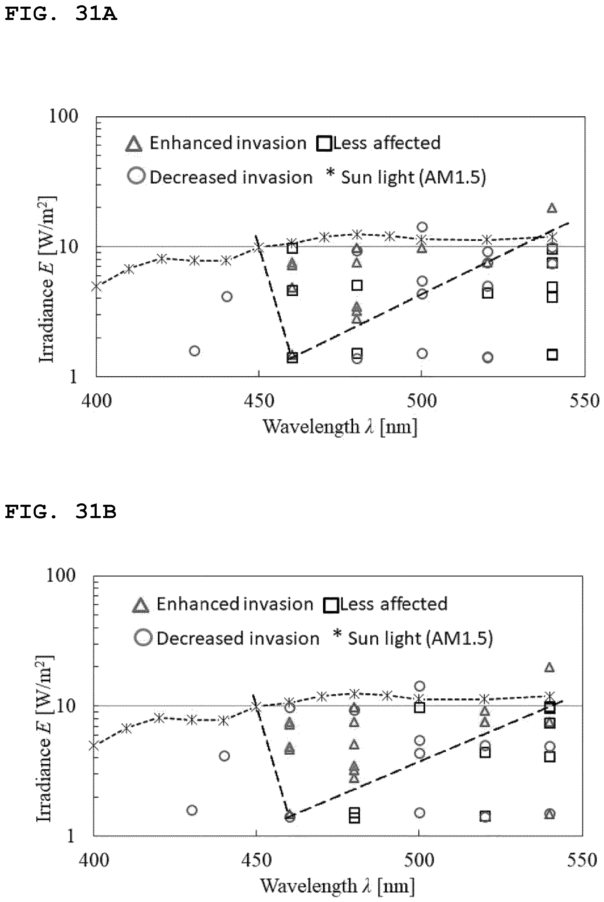

   Fig. 31A is plots of aspects of changes in the invasion of a lens epithelial cell in the relation between the irradiance (vertical axis: the unit is W/m²) and the wavelength (horizontal axis: the unit is nm) of each light source (half width: 10 nm) used in the experiments. Sunlight is also shown in the plots for reference. Fig. 31A is a view when the width w of the groove was set to 8.9 µm.
[Fig. 31B]
   Fig. 31B is plots of aspects of changes in the invasion of a lens epithelial cell in the relation between the irradiance (vertical axis: the unit is W/m²) and the wavelength (horizontal axis: the unit is nm) of each light source (half width: 10 nm) used in the experiments. Sunlight is also shown in the plots for reference. Fig. 31B is a view when the width w of the groove was set to 9.6 µm.

### [Description of Embodiments]

### [Embodiment 1]

### [Details of information]

Information that triggered the creation of one example of the present invention was obtained by the following test.

### <Cells used>

Cells that are used in one example of the present invention were selected from the following viewpoint.

First, the kinds of lens epithelial cells to be used in the test are determined to investigate a relationship between the wavelength and irradiance of light that decides whether or not the lens epithelial cells cause a change in all of the above-described steps. Hereinafter, combinations of the wavelength and irradiance of light will be referred to as "irradiation conditions." In order to solve problems of symptoms and diseases relating to human lens epithelial cells, it is preferable to acquire relationships using human primary lens epithelial cells.

However, human primary cells have the following defects: the properties vary with donors, the number of times of cell division is limited, and the properties vary with the number of times of cell division, which makes it difficult to secure the reproducibility of evaluation results (scientific problems); cells themselves and reagents and the like necessary for cell culture are expensive as a whole (economic problems); and furthermore, difficult procurement.

Therefore, it is second preferable to acquire relationships using human cell lines. Since there are various kinds of cell lines, it is more preferable to examine the adequacy with a plurality of kinds of cell lines after the initial data is obtained. In addition, at that time, furthermore, it is preferable to finally confirm the adequacy of the relationships using human primary cells. At this time, it is still more preferable to use human primary cells derived from a plurality of donors. Furthermore, preferably, it is preferable to simultaneously acquire the subject's lens epithelial cells at the time of the subject's cataract surgery and confirm the characteristics (resistance) to light.

In the case of considering easier procurement and handling and economic efficiency, it is also possible to use animal cells. Even in this case, it is preferable to examine the adequacy using human cells after the relationships are acquired. In the case of selecting the kind of the animal cells, it is preferable to select the kind having similar properties to those of humans.

Cell culture for cells selected as one example of the present invention based on the above-described viewpoint will be described below.

As the cells, human lens epithelial cells SRA01/04 (RCB1591, RIKEN BRC) were used.

Reagents are as described below.
· D-MEM (low glucose, 041-29775, Wako) + 20% fetal bovine serum (FBS, gibco, 10437028) + 100 units/mL of penicillin and 100 µg/mL of streptomycin (P/S, Gibco, 15140-122) (hereinafter, also simply referred to as "culture solution")
· Phosphate buffer solution (PBS (-) solution, Nacalai Tesque, 07269-84)
· Trypsin/EDTA solution 0.25% solution (Sigma, T4049)

The cells were sub-cultured when having become 80% to 100% confluent. A culture medium in a 60 mm dish was removed with an aspirator and washed using the PBS (-) solution. After that, 1 mL of the Trypsin/EDTA solution was added thereto, the culture medium was placed still at 37°C under 5% CO₂ for three minutes to exfoliate the cells from the dish.

The reaction of the Trypsin/EDTA solution was stopped by adding a culture medium and centrifuged for five minutes (an apparatus used was LY3690-A000, Kubota). The supernatant was removed using the aspirator, and a culture medium was added to produce a cell suspension. Cells were counted, and 5.0 × 10⁴ cells/mL of the cells were sowed in a 35 mm or 60 mm dish. The medium was exchanged every two or three days.

### <Light source used and irradiance conditions>

The content of light irradiation is as described below.

Devices used for the light irradiation will be listed below.
· LED controller (CL-1501, ASAHI SPECTRA)
· LED head 430-900B (CL-H1-430-9-1-B, ASAHI SPECTRA)
· LED head 450-900B (CL-H1-450-9-1-B, ASAHI SPECTRA)
· LED head 470-900B (CL-H1-470-9-1-B, ASAHI SPECTRA)
· LED head 505-900B (CL-H1-505-9-1-B, ASAHI SPECTRA)
· LED head 590-900B (CL-H1-590-9-1-B, ASAHI SPECTRA)
· LED head cover glass (CL-H1GCQ01, ASAHI SPECTRA)

Irradiance conditions are as described below.

Light irradiation was performed 15 cm above the cell culture surface under a cell culture environment (under an atmospheric atmosphere in which the air temperature was set to 37°C and the concentration of CO₂ was set to 5%). At that time, it was expected that the influence of the light irradiation on the cells varied with the intensity of irradiation light even when the wavelength was the same. Therefore, individual experiments were performed with the intensity changed for individual experiments with the controller while using a plurality of LED heads. The relation between the intensity of the LED controller and the irradiance at each wavelength is shown in Table 1.

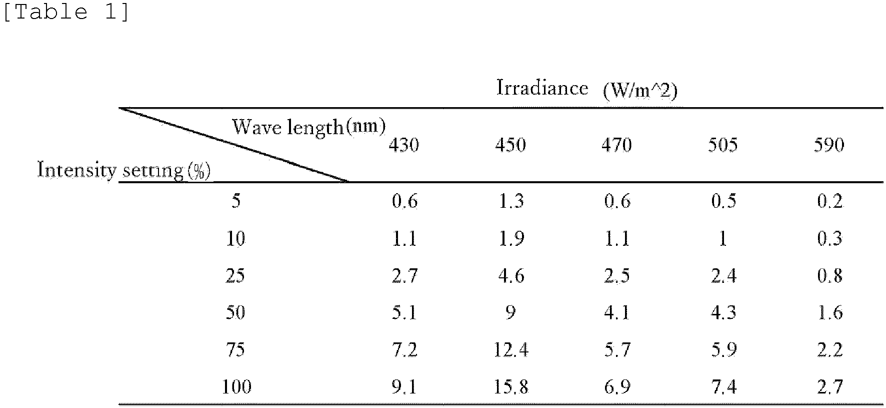

Fig. 1 is a plot showing the spectral irradiance (vertical axis: the unit is W/m²/nm) and wavelength (horizontal axis: the unit is nm) of sunlight (AM1.5 and after corneal penetration) on the front surface of a crystalline lens.

Fig. 2 is a plot showing the spectral irradiance (vertical axis: the unit is W/m²) and wavelength (horizontal axis: the unit is nm) of each LED light (half width of about 20 nm) used in the experiment. Sunlight on the front surface of the crystalline lens for each bandwidth under the same conditions is also shown in the plots for reference.

AM is referred to as air mass and is the ratio of the path of the sunlight to the path of sunlight in the case of being incident perpendicularly on the ground, which is a reference (AM1), and it is normal to employ AM1.5 in the latitude of Japan.

In some tests out of a plurality of tests, the wavelengths were commonly set to 430 nm, and only the irradiance was changed. The irradiance used is the LED controller intensities of 100% (about 9 W/m²), 75% (about 7.2 W/m²) , 50% (about 5.1 W/m²) , and 25% (about 2.7 W/m²). The light irradiation time was set to 24 hours.

In some of the different tests out of the plurality of tests, the irradiance was set to 2.4 to 2.7 W/m², and the lengths of the wavelengths were changed. The wavelengths used are five wavelengths of 430 nm, 450 nm, 470 nm, 505 nm, and 590 nm. The light irradiation time was set to 24 hours for the wavelengths of 430 nm and 450 nm and set to 48 hours for the wavelengths of 470 nm, 505 nm, and 590 nm.

### <Evaluation of cell peripheral shape>

First, the relationship 1 between the wavelength and irradiance of light that decides whether or not a morphological change of an ocular cell (hereinafter, a lens epithelial cell will be exemplified) is caused for a subject was investigated. The relationship 1 may be a plot 1 that decides whether or not a morphological change of the ocular cell is caused in the relation between the wavelength and irradiance of the light. The relationship 1 is not limited to a relationship specified in the form of a plot and may be procured as data.

In order to obtain the relationship 1, qualitative evaluation and quantitative evaluation were performed on the morphological change of the lens epithelial cell. In tests relating to the qualitative evaluation and the quantitative evaluation, cells that had become 90% to 100% confluent was irradiated with light.

### (Qualitative evaluation)

Cell membrane staining was performed after light irradiation at a predetermined wavelength and a predetermined irradiance. The supernatant was removed using an aspirator, 0.5 mL of 0.1% CellMask Orange Plasma Membrane Stains (C10045, Life Technologies) was added to a culture medium, and the culture medium was placed still for 10 minutes under an atmospheric atmosphere in which the temperature was set to 37°C and the concentration of CO₂ was set to 5%. After that, the supernatant was removed with the aspirator, 2 mL of the medium was added thereto, and similarly, the culture medium was placed still for 10 minutes under an atmospheric atmosphere in which the temperature was set to 37°C and the concentration of CO₂ was set to 5%. This operation was repeated three times. After that, the cells were observed.

In the observation of the cells, fluorescent microscopy was employed. Specifically, the stained cells were observed using an inverted microscope (FSX100, OLYMPUS) at 10 times (NA = 0.55, OLYMPUS).

Fig. 3 is photographs showing the observation results in the qualitative evaluation. The light irradiation conditions in Fig. 3(a) are a wavelength of 430 nm and an irradiance of 9.0 W/m², the light irradiation conditions in Fig. 3(b) are a wavelength of 430 nm and an irradiance of 5.1 W/m², and the light irradiation conditions in Fig. 3(c) are a wavelength of 450 nm and an irradiance of 4.6 W/m².

The lens epithelial cell in Fig. 3(a) has been fragmented and shrank. The lens epithelial cell in Fig. 3(b) has been shrank. That is, in the lens epithelial cells in Fig. 3(a) and Fig. 3(b), apoptosis has progressed. The morphology of the lens epithelial cell in Fig. 3(c) remains unchanged.

Fig. 4 is a plot in which the morphological change of the lens epithelial cell is summarized by aspect in the relation between the irradiance (vertical axis: the unit is W/m²) and the wavelength (horizontal axis: the unit is nm) of each LED light (half width of about 20 nm) used in the experiment. Sunlight on the front surface of the crystalline lens for each bandwidth under the same conditions is also shown in the plots for reference.

### (Quantitative evaluation)

A lens epithelial cell separately prepared from the lens epithelial cell used in the qualitative evaluation was cultured under the same conditions as those under which the lens epithelial cell for observation had been obtained in the qualitative evaluation, and a lens epithelial cell for the quantitative evaluation was obtained. A quantitative analysis of the cell peripheral shape was performed on this lens epithelial cell.

The shape was measured using image analysis software (ImageJ) from a stained image of the cell captured using FSX100. Thirty cells were arbitrarily selected, the contour of each cell membrane was extracted manually, and the shape was measured.

As the parameters of the measured shape, the area, circularity, and aspect ratio of the cell membrane were calculated.

Fig. 5 is a view for describing the circularity and aspect ratio of the lens epithelial cell. When the area is denoted as S, and the circumferential length is denoted as L, the circularity is indicated by 2πS/L² in the present specification. When the major axis of the lens epithelial cell elliptically approximated with ImageJ is denoted as a, and the minor axis is denoted as b, the aspect ratio is indicated by a/b in the present specification.

In Fig. 3(c) showing a normal cell, the circularity was 0.63, the aspect ratio was 1.8, and the area was 4.0 × 10⁻⁹ m².

On the other hand, in Fig. 3(a) showing a cell in which a morphological change was caused, the circularity was 0.21, the aspect ratio was 1.8, and the area was 2.1 × 10⁻⁹ m².

In Fig. 3(b) showing a cell in which a morphological change was caused, the circularity was 0.51, the aspect ratio was 1.1, and the area was 2.4 × 10⁻⁹ m².

In order to obtain the relationship between the wavelength and irradiance of light that decides whether or not a morphological change of a lens epithelial cell is caused for the subject along with the quantitative evaluation, metrics for evaluating whether or not a morphological change is caused are determined. That is, since the morphology of a cell that is used in a test varies with the kind, it is preferable to evaluate and determine the metrics for the morphological change in accordance with the kind of the cell.

As one specific example of determining the metrics, candidates of the following measurement items can be considered, and a plurality of these candidates can also be combined together.
<1> Tone scaling of microscopic image
<2> Image judgment of fluorescent staining of cell membrane and fluorescence microscopic image with computer software a. Judgement of circularity by automatic measurement of cell membrane outer circumference and cell spread area b. Judgement of aspect ratio of cell shape

In the tone scaling of the microscopic image, realistically, the microscopic image or the like is scaled into about two to five levels. In the above described <2> image judgment with software, the area and outer circumferential length of the cell and the distances between two points are measured by recognizing the difference between the cell portion and the background. At this time, measurement is performed on microscopic images in a plurality of arbitrary fields of view in consideration of a variation in the morphology of the cell.

Measurement items to be used are selected based on the results (results in the initial state of the experiment) obtained by the initial preliminary feasibility study. At that time, measurement items with which a change can be acutely perceived based on the results obtained by the initial test light irradiation condition experiment in an ordinarily-known abnormal state are selected. Furthermore, the abnormal state in the selected measurement items is defined and used as the metrics. Based on the results of the initial light irradiation condition experiment, the light irradiation time for observing a morphological change, the downtime, the observation timing and the like are also determined.

One example of determining the metrics is as described below. Table 2 below shows the invested rates of morphologically irregular cells in dark places (negative control).

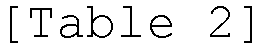

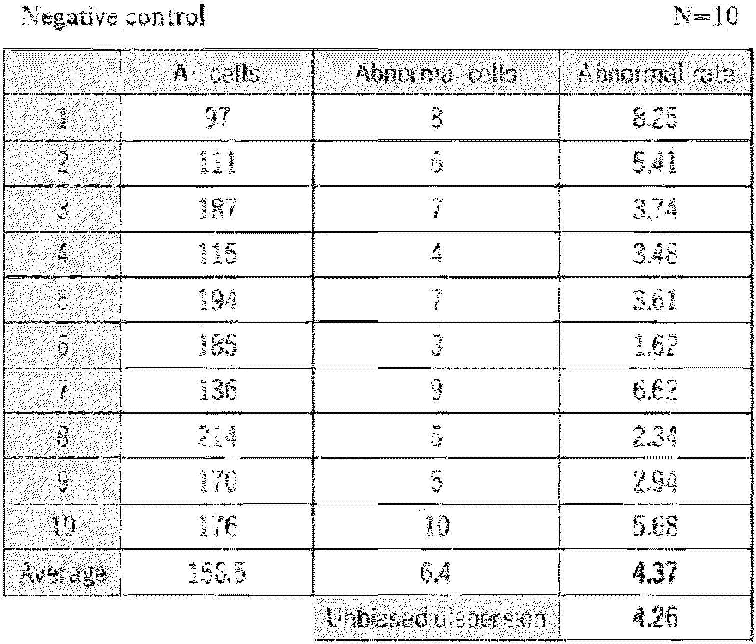

From Table 2, it is found that, even in a case where light irradiation is not performed, apoptosis or irregular morphology similar to apoptosis occurs. The rate thereof was generally 4.37% on average, and the unbiased estimate of variance was 4.26 (S2).

Therefore, in the present embodiment, when morphologically irregular cells were 20%, the state was regarded as a state different from dark place conditions, that is, a state where phototoxicity occurs. This value is a value sufficiently larger than the 5% significance level (t distribution (t = 1.833 when the degree of freedom (n - 1) is 9)) (t-test).

Next, the relationship 1 between the wavelength and irradiance of light that decides whether or not a morphological change of the lens epithelial cell is caused is clarified using the metrics. In order for that, changes in the morphology are observed at a determined timing under conditions of different wavelengths and irradiance of light, and whether or not irregular morphology has occurred at each point is plotted. Such a plot is referred to as action spectrum.

A range where irregular morphology is caused and a range where irregular morphology is not caused are roughly determined by the plot. Next, a boundary between those ranges is temporarily determined, and a boundary is determined from the group of points that are positioned in the boundary. One example of a determination method is the least squares method or the like. This boundary is a plot showing the relationship 1.

The boundary can be determined as described above since there is a principle that a stronger irregularity is caused as the wavelength of light is shorter (a chemical change of a substance is caused even when the energy of one photon having such a wavelength is large and the irradiance is low) and as the irradiance is higher.

Here, the evaluation has been described from the viewpoint of "whether or not irregular morphology is caused" compared with (based on) a state of no light irradiation; however, additionally, there are cases where there are light irradiation conditions under which a state where the morphology is estimated to be more favorable compared with that in the state of no light irradiation (a state of a favorable circularity of the cell or the like) is formed, and there is the relationship 1 under which no irregularity is caused and a more desirable state can be obtained.

Quantitative evaluation was performed using the action spectrum.

Fig. 6 is bar graphs showing the results of the quantitative evaluation, Fig. 6(a) is a graph of the area S, Fig. 6(b) is a graph of the circularity, and Fig. 6(c) is a graph of the aspect ratio.

As shown in Fig. 6, the results of the quantitative evaluation show that, as the irradiance was increased, the area of the lens epithelial cell increased in a plurality of samples, but decreased in another plurality of samples. The circularity increased as the irradiance increased.

Generally, both the cell area and the circularity decrease due to apoptosis. The aspect ratio decreases or remains constant. Therefore, a change in the area or circularity in the quantitative evaluation described in the upper paragraphs is clearly distinctive from a behavior that is caused by apoptosis, which can be generally considered.

The present inventors obtained information that an increase in the irradiance makes the active extension and contraction function of the lens epithelial cell deteriorate. The present inventors further advanced studies and investigated the relation between the irradiance and a change in the migration of the lens epithelial cell.

### <Evaluation of cell migration>

The relationship 2 between the wavelength and irradiance of light that decides whether or not the migration of the lens epithelial cell is changed is also determined by the same steps as in the relationship 1. That is, metrics are determined first using the kind of the cell that has been determined to be used in all of the steps at first, and the relationship is then studied.

First, metrics for evaluating whether or not the cell migration is changed are determined. Here, the cell migration refers to the migration distance per time when a cell migrates on a polystyrene culture surface for tissue culture in the culture solution within a certain time (hereinafter, also referred to as "migrates in the culture medium"). In order to determine the cell migration, the culture conditions for a cell under which the amount of migration of the cell can be observed or the measurement conditions of the migration distance are determined, and the cell is then observed over time to determine the cell migration per time. As an example of the culture conditions under which the amount of migration can be observed, the inside of the medium is divided into blocks with a partition or the like, and a place where the cell has been sowed and a place where the cell is not sowed (gap) are made. A linear non-sowed region having a specific width, a round non-sowed region, and the like are formed. The partition or the like is removed in such a state, the measurement begins from that time, and the movement of the cell is then observed. When the cell migration is appropriate, the cell migrates, and the cell non-sowed region (gap) decreases and is then blocked. The migration distance per time until the non-sowed region decreases and is blocked is referred to as the cell migration rate under the culture conditions.

With the study of the cell migration and the light irradiation conditions in the initial experiment, a principle that, regarding the cell migration as well, a stronger abnormality is caused by light having a wavelength shorter than normal or irradiation with a stronger intensity was observed. Therefore, as the metrics, a plot of the cell migration (cell migration distance per time) under each light irradiation condition for a specific kind of cell, a plot of the amounts (differences or rates) of the cell migration changed with no light irradiation and under specific light irradiation conditions or the like can be considered. The relationship 2 is determined by acquiring such plots. One specific example of the above-described contents is as described below.

In order to evaluate the migration of lens epithelial cells, a cell non-sowed region (gap) having a predetermined width is formed on a surface having a large number of cells. In addition, the influence of light irradiation on the migration of the cell was investigated based on the blocking process of this gap.

Up to centrifuge, the same operation as that in the section <cells used> was performed on the cells used in the present experiment. In addition, in the present experiment, 10000 cells/mL of a cell suspension was produced. After that, 700 cells were sowed in each well of a culture-insert 2 well (ib81176, ibidi) attached to the inside of a 60 mm dish in order to form a gap having a width of about 500 µm.

Fig. 7 is a schematic plan view of wells used to form a gap (scratch) having a predetermined width on a surface having a large number of cells.

The culture-insert 2 well was removed one day after the sowing of the cells, and light irradiation was initiated after the formation of the gap was confirmed. The gap was captured using the inverted microscope at a magnification of four times immediately after the initiation of the light irradiation, six hours later, 12 hours later, 24 hours later and 48 hours later, and the gap was observed. The distances between the edges of the captured gap were measured using image analysis software (ImageJ). The measurement was performed on 10 arbitrary places at each time after the light irradiation, and the average value thereof was obtained and regarded as the distance between the edges of the gap.

Fig. 8 is views showing the appearances of the blocking of the gap when the wavelength was 430 nm and the irradiance was 9.0 W/m², Fig. 8(a) is a photograph showing the appearance of the gap after 0 hours (immediately after the initiation of the light irradiation), Fig. 8(b) is a photograph showing the appearance of the gap after 24 hours from the initiation of the light irradiation, and Fig. 8(c) is a photograph showing the appearance of the gap after 48 hours from the initiation of the light irradiation.

Fig. 9 is views showing the appearances of the blocking of the gap when the wavelength was 430 nm and the irradiance was 5.1 W/m², Fig. 9(a) is a photograph showing the appearance of the gap after 0 hours (immediately after the initiation of the light irradiation), Fig. 9(b) is a photograph showing the appearance of the gap after 24 hours from the initiation of the light irradiation, and Fig. 9(c) is a photograph showing the appearance of the gap after 48 hours from the initiation of the light irradiation.

Fig. 10 is views showing the appearances of the blocking of the gap when the wavelength was 430 nm and the irradiance was 2.7 W/m², Fig. 10(a) is a photograph showing the appearance of the gap after 0 hours (immediately after the initiation of the light irradiation), Fig. 10(b) is a photograph showing the appearance of the gap after 24 hours from the initiation of the light irradiation, and Fig. 10(c) is a photograph showing the appearance of the gap after 48 hours from the initiation of the light irradiation.

Fig. 11 is views showing the appearances of the blocking of the gap when the wavelength was 470 nm and the irradiance was 2.5 W/m², Fig. 11(a) is a photograph showing the appearance of the gap after 0 hours (immediately after the initiation of the light irradiation), Fig. 11(b) is a photograph showing the appearance of the gap after 24 hours from the initiation of the light irradiation, and Fig. 11(c) is a photograph showing the appearance of the gap after 48 hours from the initiation of the light irradiation.

The size of the scale (horizontal line) in the photographs of Fig. 8 to Fig. 11 are 500 nm.

Fig. 12 is a plot in which the relation between the distance (unit: µm) between the edges of the gap and the time from the initiation of the light irradiation (horizontal axis: the unit is h) is summarized for each wavelength. The slope of the plot is distance/time and indicates the migration rate of the lens epithelial cell.

It was found that there is a correlation between the wavelength of light for irradiation and the migration rate as shown in Fig. 12.

In addition, the present inventors found from the observation of the appearance of the gap blocking in each drawing that the decrease rate of the cell migration can be divided into several groups.

Fig. 13 is a histogram showing the distribution of the decrease rates of the cell migration during the gap blocking.

As shown in Fig. 13, two steps of changes were observed regarding the decrease rate. Therefore, large decrease rates (the decrease rates of 80% or more) and intermediate decrease rates (the decrease rates of 40% to 70%) were differentiated as "large effect" and "middle effect", respectively. Hereinafter, the cell migration will be classified based on these two steps of changes as the boundary.

### <Information obtained from evaluation results of morphological change and cell migration change of lens epithelial cell>

Fig. 14 is a plot in which the aspects of the morphological changes and the aspects of the cell migration changes of lens epithelial cells are grouped together in the relation between the irradiance (vertical axis: the unit is W/m²) and wavelength (horizontal axis: the unit is nm) of each light source (half width: 10 nm) used in the experiment. Sunlight is also shown in the plots for reference.

The leftmost dotted line plot in Fig. 14 is the relationship 1 between the wavelength and irradiance of light that decides whether or not a morphological change of the ocular cell is caused for the subject, and is specifically the plot 1 that decides whether or not a morphological change of the ocular cell is caused in the relation between the wavelength and irradiance of the light.

The rightmost dotted line plot in Fig. 14 is the relationship 2 between the wavelength and irradiance of light that decides whether or not the migration of the ocular cell is changed for the subject, and is specifically a plot 2 that decides whether or not the migration of the ocular cell is changed in the relation between the wavelength and irradiance of the light.

In the relation between the wavelength and irradiance of the light, the plot 1 is on the short wavelength side of the plot 2,
in the relation between the wavelength and irradiance of the light, the plot 1 classifies a region into a plurality of regions 1 composed of a region where the morphological change is caused and a region where the morphological change is not caused, and
in the relation between the wavelength and irradiance of the light, the plot 2 classifies a region into a plurality of regions 2 composed of a region where a change (deterioration as one example) of the cell migration is caused and a region where a change of the cell migration is not caused.

The center dotted line plot in Fig. 14 is a plot 3 that further classifies the region where a change in the cell migration is caused in the regions 2 depending on the degree of the change of the cell migration. The plots 1 to 3 are approximate curves that divide the respective regions. The approximate curve may be created by selecting two points closest to the boundary between individual regions adjacent to each other from each region, and in this case, the approximate curve becomes the boundary from a total of the four points.

The plots in Fig. 14 are preferably obtained in advance before one embodiment of the present invention is carried out. That is, one example of the present invention may not include a step of creating the plots in Fig. 14 from the scratch. In addition, each plot in Fig. 14 becomes a straight line (approximate straight line) when expressed in logarithm.

What is found from Fig. 14 is that, for a certain person (subject), when light that is incident on the eye has a certain wavelength and certain irradiance, the light has a certain influence on ocular cells. As shown in Fig. 14, if no light having a short wavelength is incident on the eyes, the influence on ocular cells is extremely small. Incidentally, the fact that no light having a short wavelength is incident on the eyes can be considered as the fact that no visible light having a short wavelength is incident on the eyes.

### <One example of present invention obtained from information>

Based on the above information, in one example of the present invention, a relationship acquisition step of acquiring the relationship 1 between the wavelength and irradiance of light that decides whether or not a morphological change of the ocular cell is caused and the relationship 2 between the wavelength and irradiance of light that decides whether or not the migration of the ocular cell is changed is performed (preferably in advance) on the subject. After that, a relationship selection step of selecting at least any of the relationship 1 and the relationship 2 depending on a degree 1 that is required for the morphological change and a degree 2 that is required for the cell migration is performed. In addition, a determination step of determining a wavelength and irradiance of light to be incident on the subject's eye based on the selected relationship is performed. This determination step may also be referred to as a spectrum irradiance range determination step.

The characteristics of light that is made incident on the eyes are determined by each of the above-described steps.

Hereinafter, the relationship selection step will be described.

Each of the above-described steps made light irradiation conditions under which a cell causes a certain abnormal state plotted and relationships thereof clarified. Next, which relationship to be based on is determined to determine the characteristics of light that is made incident on the eye.

A thought that serves as the foundation of determination is whether an abnormal state that is caused in a cell is harmful or not to the subject. At a glance, it is considered to be most desirable that the abnormal state is never harmful and light under harmful irradiation conditions is fully removed. However, in actual cases, light having diverse and wide wavelength widths needs to be incident, and there is a basic principle that it is more desirable not to change ambient light unless it is obvious that the ambient light is clearly harmful in order to avoid taking a different undiscovered risk.

Originally, human bodies use light to recognize information of the external world, and it is thus considered that light having diverse and wide wavelength widths needs to be incident. In addition, the harmfulness also varies with the length of the irradiation time or the downtime.

When attention is paid to individual subjects, the harmfulness depends on individual differences of the eye optical system relating to ages and the like, the strength of the resistance of cells depending on the presence or absence of diseases, and the state at that point in time (for example, immediately after a surgery, immediately after damage or the like). Therefore, there are cases where additional studies are required as necessary regarding how much the relationship change in association with the above-described change in the conditions.

In consideration of the above-described conditions, it is determined which degree of which relationship is desirably based on in consideration of the degree 1 of the morphological change and the degree 2 that is required for the cell migration. In addition, the wavelength and irradiance of light to be incident on the eyes of the examinee are determined based on the selected relationship. In this determination, for example, which region in Fig. 14 the wavelength and irradiance of light need to belong to may be used as a reference.

There is a principle that, normally, light having a shorter wavelength and irradiation at a high intensity cause a stronger abnormality. Therefore, irradiance is plotted for each wavelength, and light to be injected is determined so that the irradiance is supposed to be the plotted irradiance or less. One specific example described hitherto in the present embodiment is based on this policy.

In a case where the degree 1 that is required for the morphological change is high and the degree 2 that is required for the cell migration is low, the region where a morphological change is not caused only needs to be selected from the regions 1. In a case where there is the plot 3, the wavelength and the irradiance in a region where the region where a change in the cell migration is caused more than the plot 3 and the region selected from the regions 1 overlap each other need to be determined as the characteristics of light that is to be made incident on the eye.

In the relationship selection step, the plot 1 and the plot 2 are preferably selected. In addition, it is preferable that a region selection step of selecting one region from the regions 1 and selecting one region from the regions 2 is further provided after the relationship selection step.

In the region selection step, it is preferable to perform at least (1) of the following (1) and (2).
(1) The region where a morphological change is not caused is selected from the regions 1.
(2) The region where a change in the cell migration is not caused is selected from the regions 2.

In addition, in the above-described determination step, the wavelength and irradiance of light commonly belonging to the individual selected regions are obtained as the wavelength and irradiance of light that is to be incident on the subject's eyes.

The plots 1 and 2 are preferably created based on regional information where the subject resides. "Regional information" may be, for example, information regarding the latitude such as AM1.5 or may be information regarding the weather. "Information regarding the weather" may be, for example, the annual average sunshine duration per day. In addition, the information may be the annual average global solar radiation or the like obtained by combining the above-described information.

The ocular cell may be a lens epithelial cell, a retinal pigment epithelial cell, a corneal endothelial cell, a corneal epithelial stem cell, or a subconjunctival fibroblast. Among them, the lens epithelial cell has high phototoxicity sensitivity and consequently enables the effect of the present invention to be particularly significantly obtained.

The technical scope of the present invention is not limited to the above mentioned embodiments, but includes various changes and modifications within a scope of deriving specific effects acquired by the constituent elements of the invention and combinations thereof.

The subject is not limited and may be a cataract patient or may not be a cataract patient.

In addition to the plot 2, the plot 3 that further classifies the region where a change of the cell migration is not caused, instead of the region where the change is caused in the regions 2, depending on the degree of the change of the cell migration may be further obtained in the relationship acquisition step.

### [Embodiment 2]

For contents that will not be described in Embodiment 2 below, the content of Embodiment 1 is employed.

### [Details of information]

In Embodiment 2, in addition to the following two relationships for the subject, which have been described in Embodiment 1,
· the relationship 1 between the wavelength and irradiance of light that decides whether or not a morphological change of the ocular cell is caused and
· the relationship 2 between the wavelength and irradiance of light that decides whether or not the migration of the ocular cell is changed,
· a relationship 3 between the wavelength and irradiance of light that decides whether or not the invasion of the ocular cell is changed
is added to the candidates of the relationships to be acquired.

In addition, in Embodiment 2, a relationship acquisition step of obtaining at least any relationship out of the relationships 1 to 3 is performed (preferably in advance). It is preferable to obtain at least two relationships out of the relationships 1 to 3 since the characteristics of more preferable light can be determined.

After that, a relationship selection step of selecting at least any of the relationship 1, the relationship 2, and the relationship 3 depending on at least any of the degree 1 that is required for the morphological change, the degree 2 that is required for the cell migration, and the degree 3 that is required for the invasion is performed. In addition, a determination step of determining the wavelength and irradiance of light that is to be incident on the subject's eyes based on the selected relationship is performed. This determination step may also be referred to as a spectrum irradiance range determination step.

The relationship 3 may be a plot 4 that decides whether or not the invasion of the ocular cell is increased in the relation between the wavelength and irradiance of the light.

In the relationship acquisition step, at least the relationship 3 may be obtained in advance, and in the relationship selection step, at least the relationship 3 may be selected. That is, the relationship 3 and the relationship 1 may be employed, or the relationship 3 and the relationship 2 may be employed.

In the relationship acquisition step, the relationships 1 to 3 may be obtained in advance, and in the relationship selection step, the relationships 1 to 3 may be selected.

The following aspects are also preferable.

"In the relation between the wavelength and irradiance of the light, the plot 4 classifies a region into a plurality of regions 4 composed of a region where an increase of the invasion is caused and a region where an increase of the invasion is not caused,
in the relationship selection step, the plot 1, the plot 2, and the plot 4 are selected,
a region selection step of selecting one region from the regions 1, selecting one region from the regions 2, and selecting one region from the regions 4 is further provided after the relationship selection step, and
in the relationship selection step, at least (1) of the following (1) to (3) is performed:
   (1) The region where the morphological change is not caused is selected from the regions 1.
   (2) The region where a change in the cell migration is not caused is selected from the regions 2.
   (3) The region where an increase of the invasion is not caused is selected from the regions 4."

In the relation between the wavelength and irradiance of the light, the plot 4 may be on the short wavelength side of the plot 3 and may be on the long wavelength side of the plot 2.

Hereinafter, invasion specific to Embodiment 2 will be described in detail. The contents regarding the morphological change and the cell migration described in Embodiment 1 may be ascertained and region-selected as the relationships together with the following content of the invasion, which is preferable.

### <Cells used>

As the cells, human lens epithelial cells SRA01/04 (RCB1591, RIKEN BRC) were used. Reagents are the culture solutions used in Embodiment 1. The cell density was set to 2 × 10⁴ cells/dish.

### <Light source used and irradiance conditions>

A device used for the light irradiation is a xenon light source (MAX-350, ASAHI SPECTRA). Light irradiation conditions are as follows.

Light irradiation was performed 15 cm above the cell culture surface under a cell culture environment (under an atmospheric atmosphere in which the air temperature was set to 37°C and the concentration of CO₂ was set to 5%). At that time, it was expected that the influence of the light irradiation on the cells varied with the intensity of irradiation light even when the wavelength was the same. Therefore, individual experiments were performed with the intensity changed for individual experiments with the controller of the light source while using a plurality of band path filters as the xenon light source. The conditions for irradiance at each wavelength are shown in Table 3. The light irradiation time was set to 24 hours.

**[Table 3]**

| Wavelength λ, mm | Irradiance E, W/m2 |
|---|---|
| NC-1, NC-2, NC-3₁, NC-4 | (~0) |
| 460 | 7.60 |
| 460 | 4.85 |
| 460 | 1.49 |
| 480 | 1.52 |
| 500 | 5.47 |
| 500 | 9.84 |
| 500 | 1.52 |
| 520 | 7.53 |
| 520 | 9.17 |
| 520 | 1.43 |
| 540 | 7.51 |
| 540 | 10.00 |
| 540 | 1.51 |
| 540 | 4.97 |

Tests under conditions of no light irradiation (negative control: NC) were each performed four times under the same conditions. The plots of the individual tests are referred to as NC-1, NC-2, NC-3, and NC-4.

### <Evaluation of invasion>

The relationship 3 between the wavelength and irradiance of light that decides whether or not the invasion of the lens epithelial cell is increased is determined by the same steps as in the relationship 1. That is, metrics are determined first using the kind of the cell that has been determined to be used in all of the steps at first, and the relationship is then studied.

First, metrics for evaluating whether or not a change in the invasion is caused are determined. Hereinafter, "invasion" in the present specification will be described.

First, a mold in which a convex stripe that is a straight line in a plan view has been patterned is prepared. Polydimethylsiloxane (PDMS) is poured into this mold, and a groove that corresponds to the convex stripe of the mold is formed in the PDMS. In addition, the PDMS is released from the mold, and a PDMS substrate on which the groove has been formed is obtained. An ocular cell is disposed in front of the groove on the PDMS substrate, and the fact that the ocular cell intrudes into the groove from the inlet of the groove (hereinafter, referred to as "opening portion") is regarded as invasion in the present specification. As shown in Fig. 15 to Fig. 18 below, a plurality of grooves having an equal width are provided on the PDMS substrate.

The following widths of grooves were prepared for each PDMS substrate. The depth of the grooves and the groove disposition intervals were set as follows.
Widths of grooves: 6.2 µm, 7.2 µm, 7.9 µm, 8.9 µm, 9.6 µm, 10.6 µm, and 11.6 µm
Depth of grooves: 10.6 µm
Groove disposition intervals: 200 µm

Cell behaviors in the opening portion of the groove were captured at 10-minutes intervals, and the invasion of the ocular cell was analyzed using image analysis software (ImageJ).

Regarding the invasion, the ocular cells were classified into the following four categories.
· Ocular cells that passed through the opening portion (showing a passing behavior) (Fig. 15)
· Ocular cells for which all of the cells intruded into the grooves and invaded (into the grooves) (Fig. 16)
· Ocular cells showing an incomplete invasion behavior (failed invasion) in which some of the cells intruded into the grooves, but crawled outside before all of the cells intruded (Fig. 17)
· Ocular cells showing invasion (crawling from grooves) in which all or part of the cells intruded into the grooves, but climbed the side walls of the grooves and crawled outside from the opening portions in the upper portions of the grooves (Fig. 18)

Fig. 15 is plan views showing the appearances of the ocular cells passing through the opening portions at the time of no light irradiation NC, Fig. 15(a) is a photograph showing the appearance after 0 hours (immediately after the initiation of light irradiation), Fig. 15(b) is a photograph showing the appearance after 10 minutes from the initiation of light irradiation, Fig. 15(c) is a photograph showing the appearance after 20 minutes from the initiation of light irradiation, Fig. 15(d) is a photograph showing the appearance after 30 minutes from the initiation of light irradiation, Fig. 15(e) is a photograph showing the appearance after 40 minutes from the initiation of light irradiation, and Fig. 15(f) is a photograph showing the appearance after 50 minutes from the initiation of light irradiation.

Fig. 16 is plan views showing the appearances of the ocular cells invading (into the grooves) at the time of no light irradiation NC, Fig. 16(a) is a photograph showing the appearance after 0 hours (immediately after the initiation of light irradiation), Fig. 16(b) is a photograph showing the appearance after 10 minutes from the initiation of light irradiation, Fig. 16(c) is a photograph showing the appearance after 20 minutes from the initiation of light irradiation, Fig. 16(d) is a photograph showing the appearance after 30 minutes from the initiation of light irradiation, Fig. 16(e) is a photograph showing the appearance after 40 minutes from the initiation of light irradiation, and Fig. 16(f) is a photograph showing the appearance after 50 minutes from the initiation of light irradiation.

Fig. 17 is plan views showing the appearances of the ocular cells performing the incomplete invasion behavior (failed invasion) at the time of no light irradiation NC, Fig. 17(a) is a photograph showing the appearance after 0 hours (immediately after the initiation of light irradiation), Fig. 17(b) is a photograph showing the appearance after 10 minutes from the initiation of light irradiation, Fig. 17(c) is a photograph showing the appearance after 20 minutes from the initiation of light irradiation, Fig. 17(d) is a photograph showing the appearance after 30 minutes from the initiation of light irradiation, Fig. 17(e) is a photograph showing the appearance after 40 minutes from the initiation of light irradiation, Fig. 17(f) is a photograph showing the appearance after 50 minutes from the initiation of light irradiation, Fig. 17(g) is a photograph showing the appearance after 60 minutes from the initiation of light irradiation and Fig. 17(h) is a photograph showing the appearance after 70 minutes from the initiation of light irradiation.

Fig. 18 is plan views showing the appearances of the ocular cells invading (and crawling from the grooves) at the time of no light irradiation NC, Fig. 18(a) is a photograph showing the appearance after 0 hours (immediately after the initiation of light irradiation), Fig. 18(b) is a photograph showing the appearance after 10 minutes from the initiation of light irradiation, Fig. 18(c) is a photograph showing the appearance after 20 minutes from the initiation of light irradiation, Fig. 18(d) is a photograph showing the appearance after 30 minutes from the initiation of light irradiation, Fig. 18(e) is a photograph showing the appearance after 40 minutes from the initiation of light irradiation, Fig. 18(f) is a photograph showing the appearance after 50 minutes from the initiation of light irradiation and Fig. 18(g) is a photograph showing the appearance after 60 minutes from the initiation of light irradiation.

Parameters used for the evaluation are as follows.
· The number of cells passing = the number of cells showing the passing behavior/the number of opening portions
· The number of cells invading (into grooves) = the number of cells for which all of the cells intrude into the grooves and invade/the number of opening portions
· The number of cells of incomplete invasion (failed invasion) = the number of cells showing the incomplete invasion behavior (failed invasion)/the number of opening portions
· The number of cells invading (crawling from grooves) = the number of cells showing the invasion behavior (crawling from grooves)/the number of opening portions

Fig. 19 is a view showing plots NC-1, NC-2, NC-3 and NC-4 wherein the number of cells invading (into grooves) is indicated along the vertical axis and the width w (µm) of the groove is indicated along the horizontal axis.

In Embodiment 2, the invasion was evaluated as follows.

In a case where the number of cells invading (into grooves) was within a range of the upper limit and the lower limit of NC at the width w of each groove, it was regarded that "the influence of light irradiation is small."

In a case where the total of the number of cells invading (into grooves) and the number of cells invading (crawling from grooves) is a higher value than the maximum value of each NC plot, the case was regarded as "increase."

In a case where the total of the number of cells invading (into grooves) and the number of cells invading (crawling from grooves) is a lower value than the minimum value of each NC plot, the case was regarded as "suppression."

The other NC plots were regarded as "low effect."

Fig. 20 is a view showing the plots NC-1, NC-2, NC-3 and NC-4 and the results at wavelengths of 430 to 460 nm when the number of cells invading (into grooves) is indicated along the vertical axis and the width w (µm) of the groove is indicated along the horizontal axis.

Fig. 21 is a view showing the plots NC-1, NC-2, NC-3 and NC-4 and the results at a wavelength of 480 nm when the number of cells invading (into grooves) is indicated along the vertical axis and the width w (µm) of the groove is indicated along the horizontal axis.

Fig. 22 is a view showing the plots NC-1, NC-2, NC-3 and NC-4 and the results at a wavelength of 500 nm when the number of cells invading (into grooves) is indicated along the vertical axis and the width w (µm) of the groove is indicated along the horizontal axis.

Fig. 23 is a view showing the plots NC-1, NC-2, NC-3 and NC-4 and the results at a wavelength of 520 nm when the number of cells invading (into grooves) is indicated along the vertical axis and the width w (µm) of the groove is indicated along the horizontal axis.

Fig. 24 is a view showing the plots NC-1, NC-2, NC-3 and NC-4 and the results at a wavelength of 540 nm when the number of cells invading (into grooves) is indicated along the vertical axis and the width w (µm) of the groove is indicated along the horizontal axis.

At the wavelengths of 430 to 460 nm (Fig. 20) and the wavelength of 480 nm (Fig. 21), tendencies of large numbers of cells invading (into grooves) compared with NC were admitted. On the other hand, at the wavelength of 500 nm (Fig. 22), the wavelength of 520 nm (Fig. 23) and the wavelength of 540 nm (Fig. 24), tendencies of large variations in the number of cells invading (into grooves) and small numbers of cells invading (into grooves) were admitted.

The following views are results obtained by arranging the numbers of cells invading (into grooves) according to the irradiance.

Fig. 25 is a view showing the results when the number of cells invading (into grooves) is indicated along the vertical axis and the width w (µm) of the groove is indicated along the horizontal axis in the case of low irradiance of 1.4 to 1.5 W/m².

Fig. 26 is a view showing the results when the number of cells invading (into grooves) is indicated along the vertical axis and the width w (µm) of the groove is indicated along the horizontal axis in the case of intermediate irradiance of 3.2 to 5.5 W/m².

Fig. 27 is a view showing the results when the number of cells invading (into grooves) is indicated along the vertical axis and the width w (µm) of the groove is indicated along the horizontal axis in the case of high irradiance of 7.5 W/m² or more.

A tendency of the number of cells invading (into grooves) decreasing as the irradiance becoming lower and increasing as the irradiance becoming higher was admitted.

The following views are results obtained by arranging the influence of light irradiation with attention paid to the number of cells passing and the number of cells of incomplete invasion.

Fig. 28 is a view showing the results when the number of cells passing is indicated along the vertical axis and the width w (µm) of the groove is indicated along the horizontal axis.

Fig. 29 is a view showing the results when the number of cells of incomplete invasion (failed invasion) is indicated along the vertical axis and the width w (µm) of the groove is indicated along the horizontal axis.

Fig. 30 is a view showing the results when the number of cells invading (crawling from grooves) is indicated along the vertical axis and the width w (µm) of the groove is indicated along the horizontal axis.

Regarding the number of cells passing (Fig. 28), the plot of 480 nm and 7.6 W/m² showed high values compared with other plots.

Regarding "failed invasion" (Fig. 29) and "crawling from grooves" (Fig. 30), the numbers were extremely low compared with the number of cells invading (into grooves) regardless of the light irradiation conditions and the width of the groove.

In consideration of the above-described contents, there is a possibility of increasing the invasion of light having a wavelength band of 480 nm or lower. Therefore, in order to prevent the increase, it is preferable to determine the characteristics of light so that the irradiance of light having a wavelength band of 480 nm or lower out of light that is incident on the subject's eyes becomes low. In particular, at a wavelength of 460 nm, even with a low irradiance of 1.49 W/m², higher invasion than in a dark place is shown, and it is thus preferable to determine the illuminance at a wavelength of 460 nm in the above-described characteristics to be sufficiently low. On the other hand, even for light having a wavelength of higher than 480 nm, at a wavelength of 540 nm, the invasion can be increased at a slightly low irradiance of 20.1 W/m², and it is thus preferable to determine the irradiance at a wavelength of 540 nm in the above-described characteristics to be low. The width w (µm) of the groove into which cells are likely to invade varies depending on the size or hardness (easiness of deformation). Therefore, the relationship between the wavelength and irradiance of light that decides whether or not the invasion is increased is preferably evaluated with respect to a plurality of grooves having a width smaller than the diameter of a cell, which is the evaluation subject, in a state where the cell has not been deformed, for example, a floating state.

### [Embodiment 3]

For contents that will not be described in Embodiment 3 below, the contents of Embodiments 1 and 2 are employed. In Embodiment 3, in addition to the human cell line (LEC, cell line) in Embodiments 1 and 2, experiment results with respect to human primary lens epithelial cells (LECs, primary) and retinal pigment epithelial cells (RPEs) will be described.

Fig. 31 is plots of aspects of changes in the invasion of a lens epithelial cell in the relation between the irradiance (vertical axis: the unit is W/m²) and the wavelength (horizontal axis: the unit is nm) of each light source (half width: 10 nm) used in the experiments. Sunlight is also shown in the plots for reference. Fig. 31A is a view when the width w of the groove was set to 8.9 µm, and Fig. 31B is a view when the width w of the groove was set to 9.6 µm. As shown by the above-described views, even when the relationship 3 is obtained in advance in the relationship acquisition step, and the relationship 3 is selected in the relationship selection step, the present invention can be established, and the problem of the present invention can be solved.

The upper side of the V-like boundary in Fig. 31 shows the increase, and the lower side shows no changes in the invasion or the suppression. The boundary is a boundary between the increase and the low effect in each NC plot and is a line obtained by the least squares method.

## Claims

1. A method of determining characteristics of light, comprising:
a relationship acquisition step of, with respect to a subject, obtaining in advance a relationship 1 between a wavelength and irradiance of light that decides whether or not a morphological change of an ocular cell is caused and a relationship 2 between the wavelength and irradiance of the light that decides whether or not migration of the ocular cell is changed;
a relationship selection step of selecting at least any of the relationship 1 and the relationship 2 depending on a degree 1 that is required for the morphological change and a degree 2 that is required for the cell migration; and
a determination step of determining a wavelength and irradiance of light to be incident on the subject's eye based on the selected relationship.

2. The method of determining characteristics of light according to claim 1,
wherein the relationship 1 is a plot 1 that decides whether or not a morphological change of the ocular cell is caused in a relation between the wavelength and irradiance of the light, and
the relationship 2 is a plot 2 that decides whether or not the migration of the ocular cell is changed in the relation between the wavelength and irradiance of the light.

3. The method of determining characteristics of light according to claim 2,
wherein, in the relation between the wavelength and irradiance of the light, the plot 1 is present on a short wavelength side of the plot 2,
in the relation between the wavelength and irradiance of the light, the plot 1 classifies a region into a plurality of regions 1 composed of a region where the morphological change is caused and a region where the morphological change is not caused,
in the relation between the wavelength and irradiance of the light, the plot 2 classifies a region into a plurality of regions 2 composed of a region where a change of the cell migration is caused and a region where a change of the cell migration is not caused,
in the relationship selection step, the plot 1 and the plot 2 are selected,
a region selection step of selecting one region from the regions 1 and selecting one region from the regions 2 is further provided after the relationship selection step,
in the relationship selection step, at least (1) of the following (1) and (2) is performed:
(1) the region where the morphological change is not caused is selected from the regions 1, and
(2) the region where a change in the cell migration is not caused is selected from the regions 2, and
in the determination step, a wavelength and irradiance of light commonly belonging to the individual selected regions are obtained as the wavelength and irradiance of the light that is to be incident on the subject's eyes.

4. The method of determining characteristics of light according to claim 3,
wherein, in addition to the plot 2, a plot 3 that further classifies the region where a change of the cell migration is caused in the regions 2 depending on a degree of the change of the cell migration is further obtained in the relationship acquisition step.

5. The method of determining characteristics of light according to any one of claims 2 to 4,
wherein the plots 1 and 2 are created based on regional information where the subject resides.

6. The method of determining characteristics of light according to any one of claims 1 to 4,
wherein the ocular cell is a lens epithelial cell, a cell retinal pigment epithelial cell, a corneal endothelial cell, a corneal epithelial stem cell, or a subconjunctival fibroblast.

7. A method of determining characteristics of light, comprising:
a relationship acquisition step of, with respect to a subject, obtaining in advance at least one of
· a relationship 1 between a wavelength and irradiance of light that decides whether or not a morphological change of an ocular cell is caused,
· a relationship 2 between the wavelength and irradiance of the light that decides whether or not migration of the ocular cell is changed, and
· a relationship 3 between the wavelength and irradiance of the light that decides whether or not invasion of the ocular cell is increased;
a relationship selection step of selecting at least any of the relationship 1, the relationship 2, and the relationship 3 depending on at least any of a degree 1 that is required for the morphological change, a degree 2 that is required for the cell migration, and a degree 3 that is required for the invasion; and
a determination step of determining a wavelength and irradiance of light that is to be incident on the subject's eyes based on the selected relationship.

8. The method of determining characteristics of light according to claim 7,
wherein the relationship 1 is a plot 1 that decides whether or not a morphological change of the ocular cell is caused in a relation between the wavelength and irradiance of the light,
the relationship 2 is a plot 2 that decides whether or not the migration of the ocular cell is changed in the relation between the wavelength and irradiance of the light, and
the relationship 3 is a plot 4 that decides whether or not the invasion of the ocular cell is increased in the relation between the wavelength and irradiance of the light.

9. The method of determining characteristics of light according to claim 8,
wherein, in the relationship acquisition step, at least the relationship 3 is obtained in advance, and in the relationship selection step, at least the relationship 3 is selected.

10. The method of determining characteristics of light according to claim 8,
wherein, in the relationship acquisition step, the relationships 1 to 3 are obtained in advance, and in the relationship selection step, the relationships 1 to 3 are selected.

11. The method of determining characteristics of light according to claim 10,
wherein, in the relation between the wavelength and irradiance of the light, the plot 1 is present on a short wavelength side of the plot 2,
in the relation between the wavelength and irradiance of the light, the plot 1 classifies a region into a plurality of regions 1 composed of a region where the morphological change is caused and a region where the morphological change is not caused,
in the relation between the wavelength and irradiance of the light, the plot 2 classifies a region into a plurality of regions 2 composed of a region where a change of the cell migration is caused and a region where a change of the cell migration is not caused,
in the relation between the wavelength and irradiance of the light, the plot 4 classifies a region into a plurality of regions 4 composed of a region where an increase of the invasion is caused and a region where an increase of the invasion is not caused,
in the relationship selection step, the plot 1, the plot 2, and the plot 4 are selected,
a region selection step of selecting one region from the regions 1, selecting one region from the regions 2, and selecting one region from the regions 4 is further provided after the relationship selection step,
in the relationship selection step, at least (1) of the following (1) to (3) is performed:
(1) the region where the morphological change is not caused is selected from the regions 1,
(2) the region where a change in the cell migration is not caused is selected from the regions 2, and
(3) the region where an increase of the invasion is not caused is selected from the regions 4, and
in the determination step, a wavelength and irradiance of light commonly belonging to the individual selected regions are obtained as the wavelength and irradiance of the light that is to be incident on the subject's eyes.

12. The method of determining characteristics of light according to claim 11,
wherein, in addition to the plot 2, a plot 3 that further classifies the region where a change of the cell migration is caused in the regions 2 depending on a degree of the change of the cell migration is further obtained in the relationship acquisition step.

13. The method of determining characteristics of light according to claim 12,
wherein, in the relation between the wavelength and irradiance of the light, the plot 4 is on the short wavelength side of the plot 3 and is on the long wavelength side of the plot 2.

14. The method of determining characteristics of light according to claim 10,
wherein at least the plots 1, 2 and 4 are created based on regional information where the subject resides.

15. The method of determining characteristics of light according to any one of claim 7 or 8,
wherein the ocular cell is a lens epithelial cell, a cell retinal pigment epithelial cell, a corneal endothelial cell, a corneal epithelial stem cell, or a subconjunctival fibroblast.
